# EUROPEAN PATENT SPECIFICATION

(11) **EP 1 704 789 B1**
(45) Date of publication and mention of the grant of the patent: **30.07.2008**
(21) Application number: 06398005.6
(22) Date of filing: 22.03.2006
(51) Int. Cl.: A43B 7/02, A43B 7/22, A43B 17/00, A43B 3/00, A61N 5/06, A61H 39/06

(54) **Postural footwear**
Haltungsschuhwerk
Chaussure de posture

(30) Priority: 22.03.2005 PT 10324805
(43) Date of publication of application: 27.09.2006
(73) Proprietor: Atlanta-Componentes Para Calcado, LDA, 4612-909 Lixa (PT)
(72) Inventor: Alves da Silva, Orlando Elisio, 2685 Portela LRS (PT)
(74) Representative: Pereira da Cruz, Jorge Afonso

(56) References cited:
- FR-A- 2 632 859
- FR-A- 2 676 918
- US-A- 5 158 526
- US-A- 5 685 094
- PATENT ABSTRACTS OF JAPAN vol. 017, no. 349 (C-1078), 2 July 1993 (1993-07-02) & JP 05 049501 A (NORIO YAZAKI), 2 March 1993 (1993-03-02)
- PATENT ABSTRACTS OF JAPAN vol. 1998, no. 04, 31 March 1998 (1998-03-31) -& JP 09 308696 A (EKOROJIKA:KK; Z WAY:KK), 2 December 1997 (1997-12-02)

## Description

### Scope of the invention

The present invention relates to postural footwear of the type consisting of a sole and an upper part, more specifically a special sole/insole providing improved walking and better static posture.

Postural footwear consists of shoes intended to improve walking and static posture. These characteristics can only be obtained in footwear that takes account of the function of the big toe, which is the toe that acts as a guide when walking and ensures good body stability. Full functionality of the big toe is only achieved when it is incorporated into the arch of the sole of the foot in order to constitute its front support.

### Prior art

Footwear designed for improving walking and static posture is known. Of the documents of prior art, we would like to mention in particular W02004/078091, AU2003262131, US 6,148,822 and FR 2 676 918.

Patent W02004/078091 relates to a sole insert to hold nutrients in/on the body, which incorporates at least one nutrient, which is capable of emitting electromagnetic waves that stimulate proprioceptive zones throughout the body, resulting in a therapeutic effect for the treatment and/or the prevention of problems connected to statural equilibrium.

Patent AU 2003262131 refers to a sole for footwear with an area on the upper surface thereof for exerting pressure on the articular receptors disposed between the astragalus and the calcaneum, and other areas which guide the movement of the foot on the physiological axis of walking. The sole also has removable correction elements which can result in an abduction or adduction and can correct a varus or valgus. For this purpose, the underside of the sole has along its inner or outer edge one or more cavities for receiving a removable correction element made of a more rigid material than the sole and with a shape appropriate for making the required correction.

Patent US 6,148,822 relates to an article such as a sole or a shoe adapted to contact the sole of a foot, which incorporates at least one unoriented mineral which is capable of developing a colour in the wavelength range between about 400 nm and 900 nm and stimulates the reflex zones located at the sole of the foot.

Patent FR 2 676 918 relates to a proprioceptive orthopaedic sole intended for re-establishing and/or maintaining correct walking dynamics, characterised in that it comprises means which, upon instigation of the step normally effected by the heel, are able to act on the muscle/joint receptors situated between the astragalus and the calcaneum, the muscle tone thus established by this initial impulse then being channelled by means guiding the movement of the foot on the physiological axis of walking.

The documents cited above take a different approach to improving walking and static posture. In fact, as well as not specifically referring to the full functionality of the big toe, which is only achieved when it is incorporated into the arch of the sole of the foot in order to constitute its front support, they do not relate to the thermal stimulation of certain areas of the foot in order to cause a reflex lowering of the distal phalanx of the big toe.

Postural footwear having polarised plates for treating reflex zones of feet is known from US 5 158 526.

### Summary of the invention

An objective of the present invention is to design footwear with a sole/insole that provides greatly improved walking and much better static posture.

Thus, the present invention uses a set of means in order to achieve the two abovementioned capacities. The means used for constructing the postural shoe are the following:
- creation of space for sagittal movement of the big toe;
- creation of an indentation for positioning the digital phalanx of the big toe;
- stimulation of the skin of the instep of the foot by means of asymmetric infrared-reflecting mirrors, in order to cause a reflex lowering of the distal phalanx of the big toe; and
- elimination of parasitic effects on posture through the creation of a shoe shape which does not cause undesired effects.

These means are not provided in prior art.

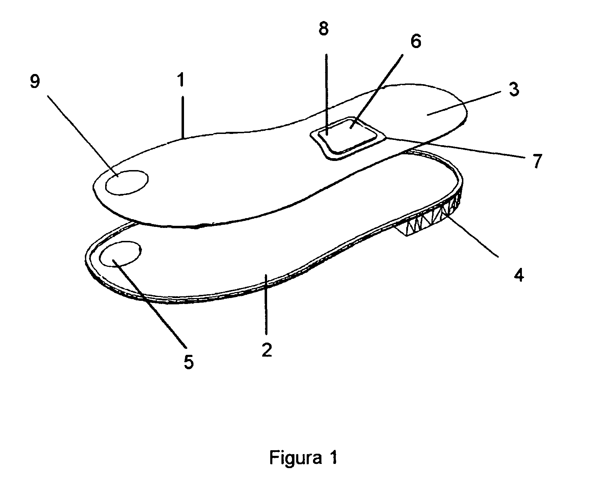

### Brief description of the drawing

The description that follows is based on the drawing attached hereto, which represents without any restrictive character a preferred embodiment. In the drawing, the single figure represents a perspective view of the sole/insole of the footwear of the invention.

### Detailed description of the invention

The sole of the foot is rich in mechanoreceptors and thermoreceptors. They provide information regarding skin deformation and pressure or temperature distensions, in addition to the information provided by the proprioceptive receptors located in the muscles and the ligaments of the ankle and the foot.

The perception of a different pressure on the surface of the sole of one foot in relation to the other is crucial for regulating balance.

A change in temperature may alter the state of contraction of the muscles of the foot, through short reflex circuits. Contact between certain parts of the foot and the ground will be altered.

The dynamics of the contact of the foot with the ground is crucial. It is particularly essential due to the contact of the big toe, which provides important information in order to ensure balance after taking a step. The receptor in the sole of the foot exerts an important action on the proprioception of the whole of the lower half of the body.

The "postural" insole stimulates the receptor in the sole of the foot by the action of the infrared-reflecting polarised plates placed in the sole region of the foot. This stimulation causes the toes to extend and separate by exciting the sole of the foot (BABINSKY phenomenon). This prevents the toes from adopting a flexed position inside the shoe when walking, which facilitates the guiding function of the big toe.

The innovation of the present invention relates to the way in which the mechanism for modifying the sensitivity of the sole of the foot is induced.

The main objective of the new postural soles/insoles is to modify the information provided to the sole of the foot by inserting two infrared-reflecting polarised plates between two layers of material forming the insole.

The innovation of the present invention lies in the asymmetric mirror effect provided by the pair of polarised plates placed in the insole, which reflect onto the skin of the foot the infrared radiations emitted by the natural heat of the foot. These radiations cause the thermal stimulation of the skin of the foot, giving rise to cyclic sensory-motor muscular reflexes which modify the contact between the ground and the foot and the ascending muscle chains.

The indentation in the digital phalanx region of the big toe is intended to position the big toe in such a way that it is in a slightly lower position than the other toes, thus facilitating its function as a guiding toe when walking.

As may be observed in the figure attached hereto, the sole/insole 1, which is a fundamental component of the postural footwear of the invention, is constituted by an actual sole 2 and an insole 3. The sole is preferably made of thermoplastic materials and the layer between the sole and the insole can be injected with an expanded material in order to improve the absorption of shocks caused by walking on uneven surfaces. The recommended maximum height of the heel 4 is 2 cm for men's shoes and 2.5 to 3 cm for ladies shoes.

Contrary to traditional footwear, this sole does not have a "cambré" (arch), i.e. its underneath part from the heel to the tip is totally flat. This helps eliminate parasitic effects on posture.

The sole 2 has an indentation 5 with a substantially elliptical configuration situated in exactly the place where the digital phalanx of the big toe rests.

The insole 3 is placed on top of the sole 2 and it has the same shape as the sole. The said insole also has a slight indentation 9 with a substantially elliptical configuration situated in exactly the place where the digital phalanx of the big toe rests, which is aligned with the indentation 5 in the sole 2

At the rear part of the foot, more specifically at a third of the distance from the heel, polarised plates 6 are placed between two lamellar pieces of leather 7 and 8. These plates stimulate the skin of the instep of the foot by means of asymmetric infrared-reflecting mirrors, in order to cause a reflex lowering of the distal phalanx of the big toe, which is an essential condition for obtaining a shoe which improves walking and postural position.

The said polarised plates 6 are made of a specific material with the particular characteristic of being capable of blocking one of the two planes of vibration of an electromagnetic wave. They are made of a long-chain polymeric material produced using a precision method which aligns the polymer molecules in a single direction.

The non-polarised light which falls on one side of the plate emerges therefrom in a parallel direction to the respective polarisation axis. In theory, the superposing of two polarised plates with perpendicular polarisation axes makes it possible to block all the incident light, meaning that also in theory this will correspond to a complete absence of transmission of incident light, thereby allowing all the energy to be used for thermal stimulation.

The invention is defined by the claims which follow.

## Claims

1. Postural footwear of the type consisting of an upper part, a sole (2) and an insole (3) wherein:
- the said sole is completely flat, without any "cambré" or arch in the part extending from the heel (4) to the tip, and has an indentation (5) with a substantially elliptical configuration situated in exactly the place where the digital phalanx of the big toe rests;
- the said insole (3) with the same shape as the sole also has a slight indentation (9) with a substantially elliptical configuration situated in exactly the place where the digital phalanx of the big toe rests, the said indentation (9) being aligned with the indentation (5) in the sole (2), and has polarised plates (6) placed between two lamellar pieces of leather (7 and 8).

2. Postural footwear according to claim 1, wherein the polarised plates (6) with a substantially trapezoidal configuration block one of the two planes of vibration of an electromagnetic wave, meaning that the non-polarised light which falls on one side of the plate emerges therefrom in a parallel direction to the respective polarisation axis, and therefore, since the said polarised plates are superposed against each other and have perpendicular polarisation axes, it is possible to block all the incident light, with a consequent complete absence of transmission of incident light, thereby allowing all the energy to be used for thermal stimulation.

3. Postural footwear according to claim 1, wherein the polarised plates (6) are placed at the rear part of the foot, at a third of the distance from the heel.

4. Postural footwear according to claim 1, wherein the heel (4) is of a maximum height of 2 cm for men's shoes and 2.5 to 3 cm for ladies' shoes.

## Patentansprüche

1. Posturales Schuhwerk bestehend aus einem Oberteil, einer Sohle (2) und einer Einlagesohle (3), wobei
- die erwähnte Sohle völlig flach ist und sich im Bereich vom Absatz (4) bis zur Fußspitze ausstreckt und keinen "cambré" (Krümmung) sondern eine Vertiefung (5) aufweist mit einer im wesentlichen elliptischen Gestaltung, welche genau auf der Stelle der Phalanx des großen Zehs sitzt;
- die erwähnte Einlegesohle (3) die dasselbe Format der Sohle hat und eine leichte Vertiefung (9) aufweist mit einer im wesentlichen elliptischen Gestaltung, welche genau auf der Stelle der Phalanx des großen Zehs sitzt, wobei diese Vertiefung (9) mit der Vertiefung (5) der Sohle (2) übereinstimmt und polarisierte Platten (6) zwischen zwei Lederlamellen (7 und 8) enthaltet.

2. Posturales Schuhwerk nach Anspruch 1, wo die polarisierten Platten, die eine im wesentlichen trapezoidalen Gestaltung haben, eines der zwei Vibrierungsebenen einer elektromagnetischen Welle blockieren, sodaß das nicht polarisierte Licht in einer paralellen Richtung in Bezug auf die betreffende Polarisierungsachse davon strahlt, wodurch es möglich ist, da die polarisierten Platten übereinander angeordnet sind und senkrechte Achsen aufweisen, das ganze darauffallendes Licht zu blockieren und, infolgedessen, die totale Absesenheit der Übertragung des darauffallendes Lichtes zu erlangen und dadurch die ganze Energie als thermische Anregung zu gebrauchen.

3. Posturales Schuhwerk nach Anspruch 1 wobei die polarisierten Platten (6) im Hinterteil des Fußes, 1/3 vom Hacken entfernt, angebracht worden sind.

4. Posturales Schuhwerk nach Anspruch 1 wobei der Schuhabsatz (4) eine maximale Höhe von 2 cm für Männerschuhe und 2,5 bis 3 cm für Frauenschuhe aufweist.

## Revendications

1. Chaussure posturale du genre de celle constituée d'une partie supérieure, d'une semelle (2) et d'une semelle intérieure (3), dans laquelle:
- ladite semelle est totalement plate, sans aucune « courbure » ou cambrure dans la partie qui s'étend du talon (4) à la pointe, ayant un creux (5) avec une configuration substantiellement elliptique situé exactement dans la zone où s'appuie la phalange digitale du gros orteil;
- ladite semelle intérieure (3) ayant la même forme que la semelle a également un léger creux (9) avec une configuration substantiellement elliptique situé exactement dans la zone où s'appuie la phalange digitale du gros orteil, ledit creux (9) étant aligné avec le creux (5) dans la semelle (2), et dispose de plaques polarisées (6) placées entres deux lamelles de cuir (7 et 8).

2. Chaussure posturale selon la première revendication, dans laquelle les plaques polarisées (6) avec une configuration substantiellement trapézoïdale bloquent l'un des deux plans de vibration d'une onde électromagnétique, ce qui fait que la lumière non polarisée qui tombe sur l'une des faces de la plaque s'en dégage dans une direction parallèle à l'axe de polarisation respectif, et donc, comme lesdites plaques polarisées sont superposées l'une contre l'autre avec des axes de polarisation perpendiculaires, il est possible de bloquer toute la lumière incidente, ayant comme conséquence l'absence totale de transmission de lumière incidente, en permettant que toute l'énergie soit utilisée pour la stimulation thermique.

3. Chaussure posturale selon la première revendication, dans laquelle les plaques polarisées (6) sont placées dans la partie postérieure du pied, à un tiers de la distance du talon.

4. Chaussure posturale selon la première revendication, dans laquelle le talon (4) est d'une hauteur maximum de 2 cm pour les chaussures pour homme et de 2,5 à 3 cm pour les chaussures pour femme.
